# EUROPEAN PATENT SPECIFICATION

(11) **EP 0 318 775 B1**
(45) Date of publication and mention of the grant of the patent: **02.02.1994**
(21) Application number: 88119211.6
(22) Date of filing: 18.11.1988
(51) Int. Cl.: C12N 15/55, C07H 21/04, C12N 1/20, C12N 9/18

(54) **A lipase gene**
Lipasegen
Gène codant pour une lipase

(30) Priority: 03.12.1987 JP 306638/87; 20.05.1988 JP 123672/88; 27.07.1988 JP 187684/88
(43) Date of publication of application: 07.06.1989
(73) Proprietor: CHISSO CORPORATION, Osaka (JP)
(72) Inventor: Aoyama, Shigeyuki, Ichiharashi Chibaken (JP); Inouye, Satoshi, Yokohamashi Kanagawaken (JP); Yoshida, Naoyuki, Ichiharashi Chibaken (JP)
(74) Representative: Kraus, Walter, Dr.

(56) References cited:
- BIOCHEMICAL AND BIOPHYSICAL RESEARCH COMMUNICATIONS, vol. 141, no. 1, 26thNovember 1986, pages 185-190, Academic Press, Inc., Duluth, MN, US; W. KUGIMIYAet al.: "Molecular cloning and nucleotide sequence of the lipase gene fromPseudomonas fragi"
- AGRICULTURAL AND BIOLOGICAL CHEMISTRY, vol. 51, no. 1, January 1987, pages 181-186, Tokyo, JP; T. NISHIO et al.: "Purification and some properties of lipaseproduced by Pseudomonas fragi 22.39 B"
- FEBS LETTERS, vol. 242, no. 1, 19th December 1988, pages 36-40, Elsevier,Amsterdam, NL; S. AOYAMA et al.: "Cloning, sequencing and expression of thelipase gene from Pseudomonas fragi IFO-12049 in E. coli"

## Description

This invention relates to a DNA arrangement coding a lipase originated from Pseudomonas genus bacterium, a recombinant DNA containing the DNA arrangement and Escherichia coli containing the same and further a process for producing the lipase.

Lipases have been used as a lipid-hydrolyzing enzyme, for oils- and fats-processing, medicine for clinical diagonosis, detergents, digestants, etc. Further, in recent years, they are important enzymes which catalyze ester hydrolysis, ester synthesis or ester conversion in the production of chemical products, particularly optically active compounds.

Further, Pseudomonas genus bacterium has been known to produce lipases useful as a catalyst for ester hydrolysis, ester synthesis or ester conversion (Japanese patent application laid-open No. Sho 62-166,898/1987).

The specific feature of the lipases produced by the Pseudomonas genus bacterium is interesting in the aspect of commercial utilization, but in order to achieve the object, a more substrate-specificity (including steric specificity) has been desired.

However, since the primary structure and higher-order structures thereof are unknown, unless the improvement is relied upon a process of inferior efficiency such as conduction of an enormous screening, conduction of a number of times of mutation treatments, etc., it has been impossible to obtain lipases having a preferred activity.

Further, since lipases are produced by expression of usually one structural gene contained in the chromosome DNA of the production bacteria, it has been difficult to improve the production of lipases with a leap.

In recent years, due to development of recombinant DNA technique, it has become possible to derive genes into those having a higher activity through its isolation or to transform nucleotide arrangement; thus it has been investigated or conducted to originate commercially interested proteins.

The present inventors have made extensive research in order to improve the above-mentioned conventional processes for modifying lipases, which processes, however, have been inferior in the efficiency.

The object of the present invention is to clone the structural gene of a lipase present in the chromosome of Pseudomonas genus bacterium to thereby determine a DNA arrangement coding the lipase, and obtain a recombinant DNA containing such a DNA arrangement and further by using Escherichia coli containing such a recombinant DNA, obtain a lipase originated from Pseudomomas genus bacterium retaining substantially the same specific features as those of the above-mentioned lipase, which lipase are utilizable for commercial use applications.

As a result, the present inventors have succeeded in cloning of the structural gene of a lipase present in the chromosome of Pseudomonas fragi IFO 12049 strain, determination of a DNA arrangement coding the lipase and production of the lipase by the medium of Escherichia coli, and have achieved the present invention.

The present invention has the following constitutions (1) to (8):
(1) A DNA arrangement coding a lipase originated from Pseudomonas genus bacterium the nucleotide of which lipase has the following arrangement: 
10 20 30 40 50 60
ATGGACGATT CGGTAAATAC ACGCTATCCG ATCTTATTGG TACACGGCCT TTTTGGATTC

70 80 90 100 110 120
GACCGGATAG GCTCGCATCA CTACTTTCAT GGTATCAAGC AAGCACTCAA TGAGTGCGGT

130 140 150 160 170 180
GCCAGCGTCT TCGTTCCAAT CATTTCAGCC GCCAACGACA ATGAAGCCCG AGGCGATCAA

190 200 210 220 230 240
CTGCTCAAGC AGATCCACAA CCTGCGCAGG CAGGTCGGTG CACAGCGTGT CAACCTGATC

250 260 270 280 290 300
GGCCACAGCC AGGGCGCCCT TACCGCGCGT TATGTGGCGG CCATCGCCCC TGAACTGATC

310 320 330 340 350 360
GCCTCGGTGA CGTCAGTCAG TGGCCCCAAC CACGGCTCCG AGCTGGCCGA TCGCTTGCGC

370 380 390 400 410 420
CTGGCCTTTG TCCCGGGGAG GCTTGGCGAA ACGGTGGCTG CCGCCCTGAC CACCTCGTTC

430 440 450 460 470 480
AGCGCATTTT TATCCGCCCT CAGCGGCCAC CCGCGCCTGC CCCAAAATGC CTTGAATGCG

490 500 510 520 530 540
CTTAACGCCC TGACCACCGA TGGGGTTGCC GCCTTCAACC GCCAATATCC CCAGGGGCTG

550 560 570 580 590 600
CCCGACAGAT GGGGCGGCAT GGGCCCCGCA CAGGTGAATG CTGTGCACTA TTATTCCTGG

610 620 630 640 650 660
AGCGGCATCA TCAAAGGCTC GCGGCTGGCC GAGTCACTCA ACCTGCTCGA CCCCTTGCAC

670 680 690 700 710 720
AATGCCTTGC GTGTGTTTGA CAGCTTTTTT ACCCGCGAAA CCCGAGAAAA CGACGGCATG

730 740 750 760 770 780
GTTGGCCGCT TCAGCTCGCA TCTGGGCCAG GTCATCCGCT CCGACTACCC TCTGGACCAC

790 800 810 820 830 840
CTCGACACCA TCAACCATAT GGCAAGAGGA AGCGCAGGCG CATCAACCCG GTAG
(2) a DNA arrangement according to item (1) wherein said Psuedomonas genus bacterium is Pseudomonas fragi IFO 12049 strain;
(3) a recombinant DNA replicable inside Escherichia coli, having a DNA arrangement coding said lipase originated from Pseudomonas genus bacterium or a DNA arrangement coding a functional equivalent to said nucleotide arrangement, each confirmed in item (1), incorporated into a vector for Escherichia coli;
(4) a recombinant DNA according to item (3), wherein said Pseudomonas genus bacterium is Pseudomonas fragi IFO 12049 strain;
(5) a recombinant DNA according to item (3), wherein said vector for Escherichia coli is pUC9;
(6) a recombinant DNA according to item (3), wherein said Pseudomonas genus bacterium is Pseudomonas fragi IFO 12049 strain and said vector for Escherichia coli is pUC9;
(7) an Escherichia coli transformed by a recombinant DNA according to item (3);
(8) an Eschericia coli transformed by a recombinant DNA according to item (3) wherein said Pseudomonas genus bacterium is Pseudomonas fragi IFO 12049;
(9) an Escherichia coli transformed by a recombinant DNA according to item (3) wherein said vector for Escherichia coli is pUC9;
(10) a process for producing a lipase which comprises using a recombinant DNA according to item (3);
(11) a process for producing a lipase which comprises cultivating an Escherichia coli according to item (7);
(12) a process for producing a lipase which comprises cultivating an Escherichia coli according to item (7) wherein said Pseudomonas genus bacterium is Pseudomonas fragi IFO 12049; and
(13) a process for producing a lipase which comprises cultivating an Escherichia coli according to item (7) wherein said vector for Escherichia coli is pUC9.

Figs. 1 to 5 each show an explanatory chart for illustrating the present invention.

Fig. 1 shows the DNA arrangement of a lipase structural gene originated from Pseudomonas fragi IFO 12049 strain.

Fig. 2 shows the restriction enzyme map of plasmid pFL-1. The portion of 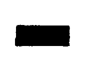
refers to a DNA fragment originated from Pseudomonas fragi IFO 12049 strain chromosome DNA.

Fig. 3 shows the respective inserted fragments of plasmids pFL-2, pFL-2SC, pFL-2ND, pFL-2AB and pFL-2SM into a plasmid pUC9 and the presence or absence of formation of clear zones in a tributyrin agar medium.

Clear zone "+" indicates formation of clear zone.

Fig. 4 shows a chromosome DNA arrangement (a linker being partly contained in a plasmid pUC9) containing a lipase structural gene of Pseudomonas fragi IFO 12049 strain of a plasmid pFL-2 and an amino acid arrangement corresponding to the lipase DNA arrangement.

Fig. 5 shows Escherichia coli JM83 (pUC9) strain (lane a) and JM83 (pFL-2) strain (lane b) analyzed with a polypeptide according to 15% SDS-polyacrylamide gel electrophoresis.

The numeral figures on the left side each show the molecular weight (×1,000) of protein marker simultaneously subjected to electrophoresis.

The recombinant DNA of the present invention is obtained by fragmenting the chromosome DNA of Pseudomonas genus bacterium producing a lipase, incorporating the resulting fragment into a multicopy type plasmid vector replicating the fragment inside Escherichia coli and selecting, from among the resulting recombinant DNAs, that containing a lipase structural gene.

The chromosome DNA of the Pseudomonas genus bacterium is prepared by subjecting the bacterial body Pseudomonas genus bacterium treated with lysozyme to lytic reaction with a proteolytic enzyme in the presence of sodium dodecylsulfate (SDS), followed by extracting the resulting lytic substance with phenol to carry out protein-removal and precipitating the DNA with ethanol.

The fragmentation of the chromosome DNA is carried out with an endonuclease having no base arrangement specificity.

Using Sau 3Al for fragmenting the Pseudomonas chromosome DNA and using pUC9 (J. Viera and J. Messing, Gene. 19, 259 (1982)) as Escherichia coli plasmid vector, it is possible to introduce the chromosome DNA fragment into the Bam Hl site as a cloning site.

Here, pUC9 is used as DNA-introducing vector for Escherichia coli, but in the present invention, any kind may be used as far as it can be replicated inside Escherichia coli, and has a suitable selective marker and the character expression of a foreign gene can be expected therefrom. For example, pUC8, pUC18, pUC19, etc. may be used.

The chromosome DNA fragment of the Pseudomonas genus bacterium decomposed with a restriction enzyme is separated according to electrophoresis in agarose gel and visualized by dyeing with ethidium bromide under ultraviolet rays and the resulting DNA fragment having a desired size is adsorbed onto a DEAE cellulose paper. The adsorbed substance is dissolved away with lM NaCℓ solution and recovered by precipitation with ethanol.

Introduction of the chromosome DNA of the Pseudomonas genus bacterium into the plasmid vector is achieved by cleaving the chromosome DNA and the plasmid vector by means of a restriction enzyme and linking both the resulting fragments together.

In advance of the linking, the cleaved vector is dephosphorylated by a bacterial alkaline phosphatase to prevent self-linking. The chromosome DNA fragment and the plasmid vector can be linked with a ligase.

Escherichia coli is transformed with the combinant DNA obtained through the above-mentioned steps and Escherichia coli having a lipase productivity is selected based on the marker of the plasmid vector and a lipase productivity.

As to Escherichia coli, as those which have an adequate marker afforded by inserting a specified cloning vector such as ampicillin-resistant bacterium selected, JM83 strain (M. Messing, R. Crea and P.H. Seeburg, Nucl. Acid. Res., 2309 (1981) may be used. Further, it is also possible to use Escherichia colis such as HB101, LE392, etc.

The transformation is carried out according to well-known competent cell method, and the selection by means of the plasmid marker is carried out by adding ampicillin (about 50 µg/mℓ) to a medium.

Further, the choice through the productivity of the lipase may be carried out by adding tributyrin (about 1%) to the medium. Namely, such a phenomenon is utilized that when the lipase is formed on the medium, a lipid is decomposed into fatty acids and the emulsified state of the lipid changes to form a circular clear zone on the periphery of the resulting colony (W. Kugiyama, Y. Otani, Y. Hashimoto and Y. Takagi, Biochem. Biophys. Res. Commun., 141, 185 (1986)).

The recombinant DNA containing the lipase structural gene obtained through the above-mentioned steps is decomposed by various restriction enzymes; a map of the restriction enzymes is prepared based on measurement according to electophoresis method using agarose gel; and subcloning of the present recombinant DNA is carried out.

Namely, when the resulting recombinant DNA is broken with a restriction enzyme; DNA fragments having a suitable size (larger than that of expected lipase structural gene, but not excessively larger than that) are collected; and these fragments are again incorporated into the plasmid vector for Escherichia coli, or the recombinant DNA is broken with a restriction enzyme; an unnecessary DNA portion is removed; and the resulting material is reunited as it is, then it is possible to reduce the molecular weight of the recombinant DNA.

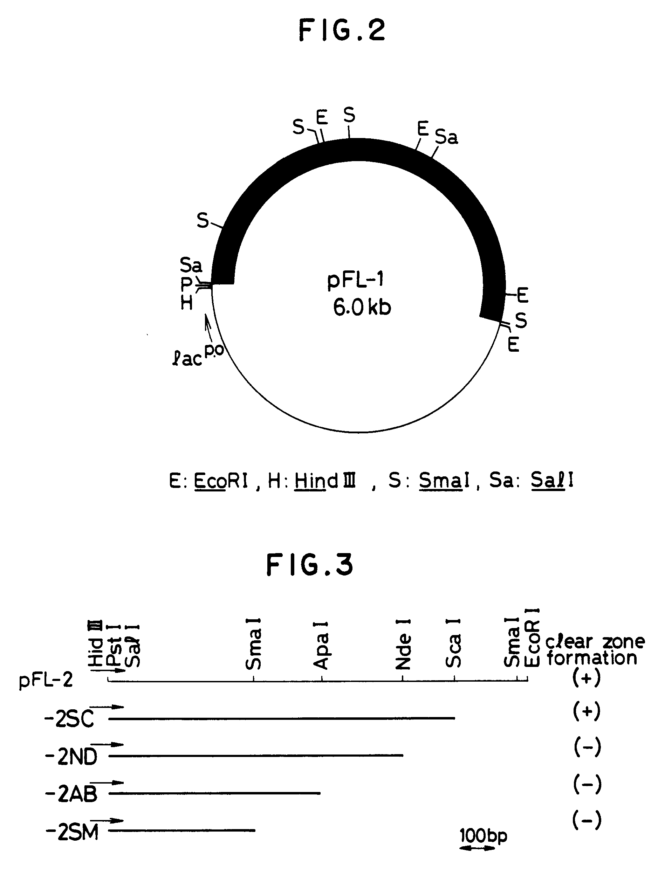

Fig. 2 shows an example of the restriction enzyme map of plasmid pFL-1 containing the lipase structural gene of Pseudomonas fragi IFO 12049 strain.

Fig. 3 shows an example of the presence or absence of formation of clear zone in a tributyrin agar medium, of Escherichia colis having the respective plasmids pFL-2, pFL-2SC, pFL-2ND, pFL-2AB and pFL-2SM, having DNA fragments broken by means of various restriction enzymes, Eco RI, Sca I, Nde I, Apa I and Sma I of plasmid pFL-1, inserted therein.

Determination of the DNA region wherein the DNA arrangement coding the lipase originated from Pseudomonas genus bacterium is present can be made according to a known method such as Sanger et al's dideoxy method (Sanger. F. et al, Proc. Natl. Acad. Sci., USA 74, 5463, 1977).

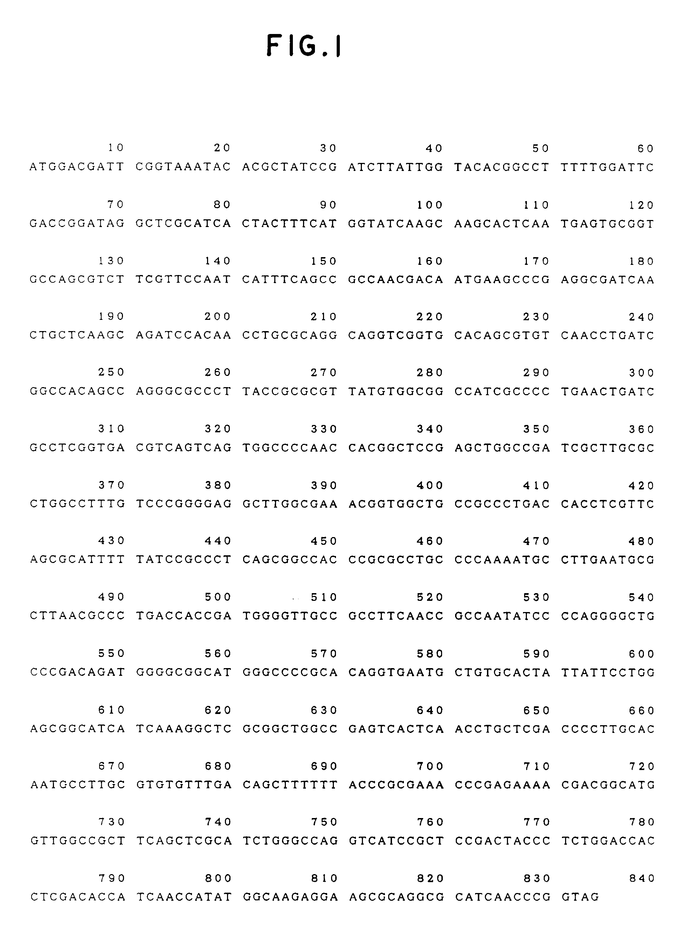

As to whether or not the DNA arrangement recited in Fig. 1 relative to the above-mentioned item (1), initiated from an initiation codon ATG and terminated by a termination codon TAG, actually corresponds to the arrangement of the lipase produced by means of Escherichia coli transformed by plasmid pFL-2, the correspondence has been judged from a fact that expression of a polypeptide having a molecular weight of ca.33,000 determined according to the above-mentioned defect experiment and SDS-polyacrylamide gel electrophoresis has been confirmed.

Namely, the DNA arrangement of the lipase structural gene of Pseudomonas IFO 12049 strain contained in the plasmid pFL-2, has been judged from a fact that the above arrangement includes an arrangement recognized by the restriction enzyme Nde I but does not include an arrangement recognized by Sca I and it is of an open reading frame wherein the molecular weight is ca. 33,000.

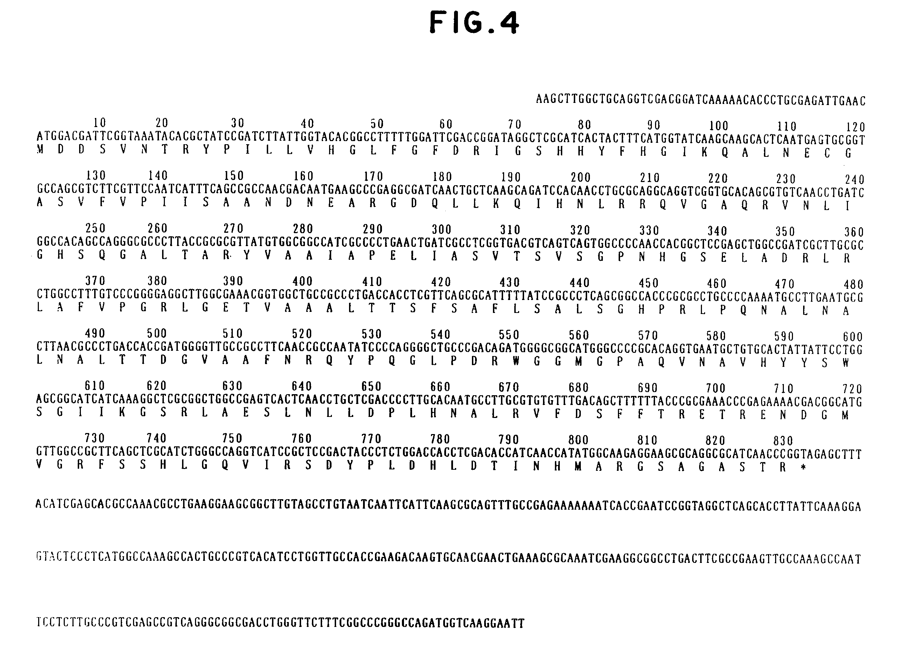

Fig. 4 shows a chart of a chromosome DNA arrangement (partly containing the linker of the plasmid pUC9) including a region wherein the lipase of the Pseudomonas fragi IFO 12049 strain of the plasmid pFL-2 is coded and an amino acid arrangement corresponding to the DNA arrangement of the lipase gene.

When Escherichia coli is transformed with a recombinant DNA containing a lipase structural gene originated from Pseudomonas genus bacterium, which recombinant DNA has been reduced in the molecular weight at the above-mentioned subcloning step, then it is possible to again obtain Escherichia coli capable of producing the lipase.

The constituent of the Escherichia coli capable of producing the lipase is separated up to a polypeptide level according to electrophoresis in SDS-polyacrylamide gel. The thus separated polypeptide is dyed in Coomassie Brilliant Blue and visualized.

Expression in Escherichia coli, of the lipase structural gene originated from Pseudomonas genus bacterium, which gene has been incorporated into a vector for introducing DNA for Escherichia coli, is confirmed by cultivating the above Escherichia coli and an Escherichia coli having no heterogeneous structural gene incorporated therein and containing only the above-mentioned vector for introducing DNA for Escherichia coli, respectively; separating the thus prepared samples into polypeptides; visualizing them; and then comparing the resulting materials.

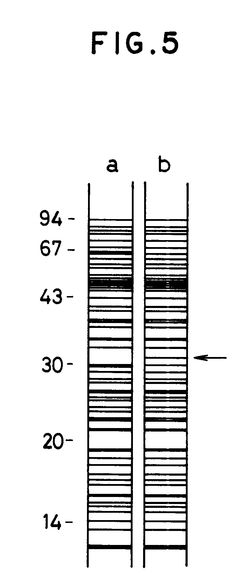

For example, Fig. 5 shows a view of polypeptides separated in SDS-polyacrylamide gel and visualized, with Escherichia coli JM83 (pUC9) strain ("a" in Fig. 5) and Escherichia coli JM83 (pFL-2) strain ("b" in Fig. 5).

By comparing the above two samples, expression in Escherichia coli, of the lipase structural gene of Pseudomonas fragi IFO 12049 strain originated from plasmid pFL-2 is confirmed.

Further, in order to ensure as high an expression level as possible, of the heterogeneous structural gene incorporated into the Escherichia coli, it is necessary to relate the above-mentioned gene to as effective a promotor-operator system as possible.

In the case of Escherichia coli, various effective promotor-operator systems have been known such as lactose (lac) operon, tryptophan (trp) operon, outer membrane protein gene (ℓpp) promotor, β-lactamase promotor, etc.

These various methods may be employed for expression in Escherichia coli within a region wherein the lipase of the present invention is coded. Further, in order to obtain similar results, various replication-initiating sites, marker genes, effective promotors and other structural elements may be combined.

Transformed Escherichia colis obtained in the above-mentioned various cases, too, are within the scope of the present invention.

Further, the reproduction conditions may be determined depending on the specific features of Escherichia coli strains so that the lipase can be reproduced most efficiently.

The present invention will be described in more detail by way of Examples, but it should not be construed to be limited thereto.

All of the restriction enzymes used in Examples are commercially available products made by NIPPON GENE CO., LTD.

### Example 1

### (Preparation of Pseudomonas fragi IFO 12049 strain chromosome DNA)

Pseudomonas fragi IFO 12049 strain was cultivated in L-medium (containing Bactotryptone (1%), a yeast powder (0.5%) and NaCℓ (0.5%) and adjusted to pH 7.2) (200 mℓ) at 30°C overnight, followed by collecting the resulting bacterial bodies, once washing the bacterial bodies with 10 mM-Tris-HCℓ buffer solution(pH 8.0) and 30-mM NaCℓ solution (10 mℓ) and suspending them in 10 mM-Tris-HCℓ buffer solution (pH 8.0) and 1mM-EDTA 2Na solution (12 mℓ), based on 100 mℓ of the culture solution.

To the resulting suspension were added Tris-HCℓ buffer solution, tris-HCl, EDTA-2Na buffer solution (pH 8.0) and lysozyme (made by Seikagaku Kogyo Company) so that their final concentrations could be 50 mM, 50 mM, 50 mM and 1 mg/mℓ, respectively, followed by allowing the resulting solution to stand at room temperature (25°C) for one hour.

To the solution was added Proteinase K (trademark of product made by Merck Company) in the presence of 0.5% SDS so as to give a concentration of 100 µg/mℓ, followed by slowly shaking the mixture at 50°C for 3 hours to dissolve the bacterial bodies.

The resulting sample was twice extracted with an equal quantity of Tris-saturated phenol, followed by precipitating the extract with ethanol, dissolving the precipitates in 10 mM Tris-HCℓ buffer solution (pH 8.0)-1mM-EDTA solution (hereinafter abbreviated to TE solution), and subjecting the solution to dialysis at 4°C for 2 days to prepare a chromosome DNA.

### Example 2

### (Cloning of lipase gene)

Pseudomonas fragi IFO 12049 strain chromosome DNA (about 7 µg) prepared in Example 1 was partially digested with lU Sau 3AI at 37°C according to reactions with lapse of times of 5, 15, 25, 35 and 50 minutes, followed by stopping the reactions with EDTA 2Na solution so as to give a final concentration of 20 mM and mixing the respective reaction solutions.

The resulting sample obtained by the mixing was subjected to electrophoresis with 40 mA for 16 hours in 0.7% agarose gel in 40 mM Tris, 2 mM sodium acetate and 1 mM EDTA 2Na, at pH 7-9.

DNA fragments of 2-6 Kb in the agarose gel were adsorbed onto whatman DE81 DEAE cellulose paper (trade-name of product made by Whatman Company), followed by dissolving out the adsorbed substance with 300 µℓ lM-NaCℓ-TE solution, twice extracting the resulting dissolved-out substance with an equal quantity of 300 µℓ lM-NaCℓ-TE solution and adding ethanol in 2.5 times the quantity of the extract to precipitate and recover it.

The quantity of the substance recovered at that time was 0.7 µg. This sample and pUC9 (1.5 µg) obtained by digesting Bam HI, followed by dephosphorizing the resulting substance with 0.6 U alkaliphosphatase (made by Takara Shuzo Company) at 50°C for 3 hours were linked by means of T4 DNA ligase of 1 µℓ (147 U/µℓ) in the presence of Tris-HCℓ buffer solution (pH 8.0) (66 mM), MgCℓ₂ (6.6 mM), ATP (0.066 mM) and dithiothreitol (10 mM) at 4°C for 16 hours.

Using the thus linked DNA sample, JM 83 strain of ∼2×10⁹ bacterial bodies was transformed according to competent cell method, followed by cultivating the resulting substance for 16 hours on L-agar medium containing Ampicillin (trademark of product made by Meiji Seika Company) (50 µg/mℓ), tributyrin (1%) and X-gal (0.0002%).

Among about 1500 transformed strains selected by marker of pUC9 plasmid vector, there was one strain which formed a clear zone in the medium. Thus this strain was separated and named Escherichia coli JM 83 (pFL-1).

### Example 3

### (Analysis of recombinant DNA)

Plasmid pFL-1 was digested with various restriction enzymes, followed by preparing a restriction enzyme map based on measurement according to agarose gel electrophoresis. The restriction enzyme map of pFL-1 is shown in Fig. 2.

Based on the restriction enzyme map of plasmid pFL-1, the respective DNA fragments obtained by breakage with the various restriction enzymes were subcloned into pUC9 to transform Escherichia coli JM 83 strain, followed by observing the presence or absence of formation of clear zone in a medium containing tributyrin (1%) as in the case of Example 2.

Fig. 3 shows the presence or absence of formation of clear zone of Escherichia colis having plasmids i.e. pFL-2, pFL-2SC, pFL-2ND, pFL-2AB and pFL-2SM, having DNA fragments obtained by breaking plasmid pFL-1 with various restriction enzymes i.e. Eco RI, Sca I, Nde I, Apa I or Sma I, inserted into the respective plasmids.

### Example 4

### (Determination of DNA arrangement)

As to the portion originated from the chromosome DNA of Pseudomonas fragi IFO 12049 strain of plasmid pFL-2, DNA arrangement was determined with M13 Sequencing Kit (trademark of product made by Takara Shuzo Company) and Deaza Sequencing Kit (trademark of product made by NIPPON GENE CO., LTD.). As a result, an open reading frame shown in Fig. 4 was confirmed.

### Example 5

### (Expression of recombinant DNA in Escherichia coli)

Escherichia colis JM 83 (pUC9) strain and JM 83 (pFL-2) strain were cultivated in a shaking culture machine at 37° for 16 hours, in a L liquid medium containing 50 µg/mℓ of Ampicillin (trademark of product made by Meiji Seika Company). The resulting bacterial bodies were collected, followed by again suspending the bodies in 0.1 M phosphoric acid buffer solution (pH 0.7), mixing the bacterial bodies corresponding to the culture solution (30 µℓ) with an equal quantity of 2X sample buffer solution (10% glycerol, 5% 2-mercaptoethanol, 2.3% SDS and 0.0625 M Tris-HCℓ (pH 6.8), boiling the mixture for 5 minutes and analyzing with polypeptide according to 15% SDS-polyacrylamide gel electrophoresis.

The SDS-polyacrylamide gel electrophoresis was carried out according to Laemmli et al's method (Laemmli, U.K. Nature 227 680 (1970)), and the electrophoresis was carried out with 20 mA till Bromophenol Blue reached the lower end of the gel.

Fig. 5 shows polypeptides relative to Escherichia colis JM 83 (pUC9) strain (lane a) and JM 83 (pFL-2) strain (lane b) dyed by Coomassie Brilliant Blue solution in 15% SDS-polyacrylamide gel. Expression (polypeptide shown by an arrow mark (←)) in Escherichia coli JM 83 strain of a lipase structural gene originated from Pseudomonas fragi IFO 12049 strain contained in plasmid pFL-2 was confirmed.

## Claims

1. A DNA arrangement coding a lipase originated from Pseudomonas genus bacterium the nucleotide sequence of which lipase has the following arrangement:

10 20 30 40 50 60
ATGGACGATT CGGTAAATAC ACGCTATCCG ATCTTATTGG TACACGGCCT TTTTGGATTC

70 80 90 100 110 120
GACCGGATAG GCTCGCATCA CTACTTTCAT GGTATCAAGC AAGCACTCAA TGAGTGCGGT

130 140 150 160 170 180
GCCAGCGTCT TCGTTCCAAT CATTTCAGCC GCCAACGACA ATGAAGCCCG AGGCGATCAA

190 200 210 220 230 240
CTGCTCAAGC AGATCCACAA CCTGCGCAGG CAGGTCGGTG CACAGCGTGT CAACCTGATC

250 260 270 280 290 300
GGCCACAGCC AGGGCGCCCT TACCGCGCGT TATGTGGCGG CCATCGCCCC TGAACTGATC

310 320 330 340 350 360
GCCTCGGTGA CGTCAGTCAG TGGCCCCAAC CACGGCTCCG AGCTGGCCGA TCGCTTGCGC

370 380 390 400 410 420
CTGGCCTTTG TCCCGGGGAG GCTTGGCGAA ACGGTGGCTG CCGCCCTGAC CACCTCGTTC

430 440 450 460 470 480
AGCGCATTTT TATCCGCCCT CAGCGGCCAC CCGCGCCTGC CCCAAAATGC CTTGAATGCG

490 500 510 520 530 540
CTTAACGCCC TGACCACCGA TGGGGTTGCC GCCTTCAACC GCCAATATCC CCAGGGGCTG

550 560 570 580 590 600
CCCGACAGAT GGGGCGGCAT GGGCCCCGCA CAGGTGAATG CTGTGCACTA TTATTCCTGG

610 620 630 640 650 660
AGCGGCATCA TCAAAGGCTC GCGGCTGGCC GAGTCACTCA ACCTGCTCGA CCCCTTGCAC

670 680 690 700 710 720
AATGCCTTGC GTGTGTTTGA CAGCTTTTTT ACCCGCGAAA CCCGAGAAAA CGACGGCATG

730 740 750 760 770 780
GTTGGCCGCT TCAGCTCGCA TCTGGGCCAG GTCATCCGCT CCGACTACCC TCTGGACCAC

790 800 810 820 830 840
CTCGACACCA TCAACCATAT GGCAAGAGGA AGCGCAGGCG CATCAACCCG GTAG

2. A DNA arrangement according to claim 1 wherein said Psuedomonas genus bacterium is Pseudomonas fragi IFO 12049 strain.

3. A recombinant DNA replicable inside Escherichia coli, having a DNA arrangement coding said lipase originated from Pseudomonas genus bacterium or a DNA arrangement coding a functional equivalent to said nucleotide arrangement, each confirmed in claim 1 , incorporated into a vector for Escherichia coli.

4. A recombinant DNA according to claim 3 , wherein said Pseudomonas genus bacterium is Pseudomonas fragi IFO 12049 strain.

5. A recombinant DNA according to claim 3 , wherein said vector for Escherichia coli is the known vector pUC9.

6. A recombinant DNA according to claim 3 , wherein said Pseudomonas genus bacterium is Pseudomonas fragi IFO 12049 strain and said vector for Escherichia coli is said pUC9.

7. An Escherichia coli transformed by a recombinant DNA according to claim 3.

8. An Eschericia coli transformed by a recombinant DNA according to claim 3 wherein said Pseudomonas genus bacterium is Pseudomonas fragi IFO 12049.

9. An Escherichia coli transformed by a recombinant DNA according to claim 3 wherein said vector for Escherichia coli is said pUC9.

10. A process for producing a lipase which comprises using a recombinant DNA according to claim 3.

11. A process for producing a lipase which comprises cultivating an Escherichia coli according to claim 7.

12. A process for producing a lipase which comprises cultivating an Escherichia coli according to claim 7 wherein said Pseudomonas genus bacterium is Pseudomonas fragi IFO 12049.

13. A process for producing a lipase which comprises cultivating an Escherichia coli according to claim 7 wherein said vector for Escherichia coli is pUC9.

## Patentansprüche

1. DNA-Anordnung, codierend eine Lipase aus einem Bakterium der Gattung Pseudomonas, wobei die Nucleotidsequenz dieser Lipase die folgende Anordnung besitzt:

10 20 30 40 50 60
ATGGACGATT CGGTAAATAC ACGCTATCCG ATCTTATTGG TACACGGCCT TTTTGGATTC

70 80 90 100 110 120
GACCGGATAG GCTCGCATCA CTACTTTCAT GGTATCAAGC AAGCACTCAA TGAGTGCGGT

130 140 150 160 170 180
GCCAGCGTCT TCGTTCCAAT CATTTCAGCC GCCAACGACA ATGAAGCCCG AGGCGATCAA

190 200 210 220 230 240
CTGCTCAAGC AGATCCACAA CCTGCGCAGG CAGGTCGGTG CACAGCGTGT CAACCTGATC

250 260 270 280 290 300
GGCCACAGCC AGGGCGCCCT TACCGCGCGT TATGTGGCGG CCATCGCCCC TGAACTGATC

310 320 330 340 350 360
GCCTCGGTGA CGTCAGTCAG TGGCCCCAAC CACGGCTCCG AGCTGGCCGA TCGCTTGCGC

370 380 390 400 410 420
CTGGCCTTTG TCCCGGGGAG GCTTGGCGAA ACGGTGGCTG CCGCCCTGAC CACCTCGTTC

430 440 450 460 470 480
AGCGCATTTT TATCCGCCCT CAGCGGCCAC CCGCGCCTGC CCCAAAATGC CTTGAATGCG

490 500 510 520 530 540
CTTAACGCCC TGACCACCGA TGGGGTTGCC GCCTTCAACC GCCAATATCC CCAGGGGCTG

550 560 570 580 590 600
CCCGACAGAT GGGGCGGCAT GGGCCCCGCA CAGGTGAATG CTGTGCACTA TTATTCCTGG

610 620 630 640 650 660
AGCGGCATCA TCAAAGGCTC GCGGCTGGCC GAGTCACTCA ACCTGCTCCA CCCCTTGCAC

670 680 690 700 710 720
AATGCCTTGC GTGTGTTTGA CAGCTTTTTT ACCCGCGAAA CCCGAGAAAA CGACGGCATG

730 740 750 760 770 780
GTTGGCCGCT TCAGCTCGCA TCTGGGCCAG GTCATCCGCT CCGACTACCC TCTGGACCAC

790 800 810 820 830 840
CTCGACACCA TCAACCATAT GGCAAGAGGA AGCGCAGGCG CATCAACCCG GTAG

2. DNA-Anordnung nach Anspruch 1, dadurch **gekennzeichnet**, daß das Bakterium der Gattung Pseudomonas der Pseudomonas fragi IFO 12049 Stamm ist.

3. Rekombinante DNA die in Escherichia coli replizierbar ist, bei der eine DNA-Anordnung, die die Lipase mit Ursprung aus einem Bakterium der Gattung Pseudomonas codiert, oder eine DNA-Anordnung, die ein funktionelles Äquivalent zu dieser Nucleotidanordnung jeweils nach Anspruch 1 codiert, in einem Vektor für Escherichia coli eingebaut ist.

4. Rekombinante DNA nach Anspruch 3, dadurch **gekennzeichnet**, daß das Bakterium der Gattung Pseudomonas der Pseudomonas fragi IFO 12049 Stamm ist.

5. Rekombinante DNA nach Anspruch 3, dadurch **gekennzeichnet**, daß der Vektor für Escherichia coli der bekannte Vektor pUC9 ist.

6. Rekombinante DNA nach Anspruch 3, dadurch **gekennzeichnet**, daß das Bakterium der Gattung Pseudomonas der Pseudomonas fragi IFO 12049 Stamm ist, und der Vektor für Escherichia coli der genannte pUC9 ist.

7. Escherichia coli, transformiert durch eine rekombinante DNA nach Anspruch 3.

8. Escherichia coli, transformiert durch eine rekombinante DNA nach Anspruch 3, worin das Bakterium der Gattung Pseudomonas Pseudomonas fragi IFO 12049 ist.

9. Escherichia coli, transformiert durch eine rekombinante DNA nach Anspruch 3, dadurch **gekennzeichnet**, daß der Vektor für Escherichia coli der genannte pUC9 ist.

10. Verfahren zur Produktion einer Lipase, dadurch **gekennzeichnet**, daß man eine rekombinante DNA nach Anspruch 3 verwendet.

11. Verfahren zur Erzeugung einer Lipase, dadurch **gekennzeichnet**, daß man Escherichia coli nach Anspruch 7 züchtet.

12. Verfahren zur Produktion einer Lipase, dadurch **gekennzeichnet**, daß man Escherichia coli nach Anspruch 7, worin das Bakterium der Gattung Pseudomonas Pseudomonas fragi IFO 12049 ist, züchtet.

13. Verfahren zur Produktion einer Lipase, dadurch **gekennzeichnet**, daß man Escherichia coli nach Anspruch 7 züchtet, wobei der Vektor für Escherichia coli pUC9 ist.

## Revendications

1. Arrangement d'ADN codant pour une lipase provenant d'une bactérie de genre Pseudomonas, la séquence nucléotidique de cette lipase ayant l'arrangement suivant :

10 20 30 40 50 60
ATGGACGATT CGGTAAATAC ACGCTATCCG ATCTTATTGG TACACGGCCT TTTTGGATTC

70 80 90 100 110 120
GACCGGATAG GCTCGCATCA CTACTTTCAT GGTATCAAGC AAGCACTCAA TGAGTGCGGT

130 140 150 160 170 180
GCCAGCGTCT TCGTTCCAAT CATTTCAGCC GCCAACGACA ATGAAGCCCG AGGCGATCAA

190 200 210 220 230 240
CTCCTCAAGC AGATCCACAA CCTGCGCAGG CAGGTCGGTG CACAGCGTGT CAACCTGATC

250 260 270 280 290 300
GGCCACAGCC AGGGCGCCCT TACCGCGCGT TATGTGGCGG CCATCGCCCC TGAACTGATC

310 320 330 340 350 360
GCCTCGGTGA CGTCAGTCAG TGGCCCCAAC CACGGCTCCG AGCTGGCCGA TCGCTTGCGC

370 380 390 400 410 420
CTGGCCTTTG TCCCGGGGAG GCTTGGCGAA ACGGTGGCTG CCGCCCTGAC CACCTCGTTC

430 440 450 460 470 480
AGCGCATTTT TATCCGCCCT CAGCGGCCAC CCGCGCCTGC CCCAAAATGC CTTGAATGCG

490 500 510 520 530 540
CTTAACGCCC TGACCACCGA TGGGGTTGCC GCCTTCAACC GCCAATATCC CCAGGGGCTG

550 560 570 580 590 600
CCCGACAGAT GGGGCGGCAT GGGCCCCGCA CAGGTGAATG CTGTGCACTA TTATTCCTGG

610 620 630 640 650 660
AGCGGCATCA TCAAAGGCTC GCGGCTGGCC GAGTCACTCA ACCTGCTCGA CCCCTTGCAC

670 680 690 700 710 720
AATGCCTTGC GTGTGTTTGA CAGCTTTTTT ACCCGCGAAA CCCGAGAAAA CGACGGCATG

730 740 750 760 770 780
GTTGGCCGCT TCAGCTCGCA TCTGGGCCAG GTCATCCGCT CCGACTACCC TCTGGACCAC

790 800 810 820 830 840
CTCGACACCA TCAACCATAT GGCAAGAGGA AGCGCAGGCG CATCAACCCG GTAG

2. Arrangement d'ADN suivant la revendication 1, dans lequel cette bactérie de genre Pseudomonas est la souche de Pseudomonas fragi IFO 12049.

3. ADN recombinant pouvant être répliqué dans Escherichia coli, ayant un arrangement d'ADN codant pour cette lipase provenant d'une bactérie de genre Pseudomonas ou un arrangement d'ADN codant pour un équivalent fonctionnel de cet arrangement nucléotidique, chacun étant tel que défini dans la revendication 1, incorporé dans un vecteur pour Escherichia coli.

4. ADN recombinant suivant la revendication 3, dans lequel cette bactérie de genre Pseudomonas est la souche de Pseudomonas fragi IFO 12049.

5. ADN recombinant suivant la revendication 3, dans lequel ce vecteur pour Escherichia coli est le vecteur connu pUC9.

6. ADN recombinant suivant la revendication 3, dans lequel cette bactérie de genre Pseudomonas est la souche de Pseudomonas fragi IFO 12049 et ce vecteur pour Escherichia coli est ce pUC9.

7. Escherichia coli transformé par un ADN recombinant suivant la revendication 3.

8. Escherichia coli transformé par un ADN recombinant suivant la revendication 3, dans lequel cette bactérie de genre Pseudomonas est la souche de Pseudomonas fragi IFO 12049.

9. Escherichia coli transformé par un ADN recombinant suivant la revendication 3, dans lequel ce vecteur pour Escherichia coli est ce pUC9.

10. Procédé pour produire une lipase, qui comprend l'utilisation d'un ADN recombinant suivant la revendication 3.

11. Procédé pour produire une lipase, qui comprend la culture d'un Escherichia coli suivant la revendication 7.

12. Procédé pour produire une lipase, qui comprend la culture d'un Escherichia coli suivant la revendication 7, dans lequel cette bactérie de genre Pseudomonas est la souche de Pseudomonas fragi IFO 12049.

13. Procédé pour produire une lipase, qui comprend la culture d'un Escherichia coli suivant la revendication 7, dans lequel ce vecteur pour Escherichia coli est pUC9.
